# EUROPÄISCHE PATENTSCHRIFT

(11) **EP 2 481 309 B1**
(45) Veröffentlichungstag und Bekanntmachung des Hinweises auf die Patenterteilung: **27.01.2016**
(21) Anmeldenummer: 11075018.9
(22) Anmeldetag: 01.02.2011
(51) Int. Cl.: A41B 11/00, A41D 13/005, A43B 7/04, H05B 3/34, A61F 7/00

(54) **Elektrisch beheizbare Socke, Sockenheizanordnung sowie Verfahren zum Herstellen einer elektrisch beheizbaren Socke**
Electrically heatable sock, sock heating assembly and method for producing an electrically heatable sock
Chaussette pouvant être chauffée électriquement, agencement de chauffage de chaussettes et procédé de fabrication d'une chaussette pouvant être chauffée électriquement

(43) Veröffentlichungstag der Anmeldung: 01.08.2012
(73) Patentinhaber: Lenz GmbH, 6858 Schwarzach (AT)
(72) Erfinder: Macher, David, 8570 Voitsberg (AT); Kremer, Gerhard, 8280 Fürstenfeld (AT); Maron, Urs, 2560 Nidau (CH)
(74) Vertreter: Pfenning, Meinig & Partner mbB

(56) Entgegenhaltungen:
- CN-U- 2 043 456
- CN-Y- 201 216 174
- DE-A1- 1 615 176
- KR-B1- 100 316 136
- US-A- 4 800 867
- US-A1- 2009 056 107

## Beschreibung

Die Erfindung betrifft eine elektrisch beheizbare Socke mit einem Fußteil und einem daran anschließenden Beinteil, wobei mindestens ein Heizelement am Fußteil angeordnet ist, das über Zuleitungen mit Anschlüssen für eine eine Batterie aufweisende Spannungsversorgung verbunden ist, wobei die Anschlüsse im oberen Bereich des Beinteils angeordnet sind. Weiterhin betrifft die Erfindung eine Sockenheizanordnung mit einer elektrisch beheizbaren Socke und einer Batterie sowie ein Verfahren zum Herstellen einer elektrisch beheizbaren Socke.

Derartige Socken, wie sie beispielsweise aus der Druckschrift DE 16 15 176 bekannt sind, weisen zumeist eine relativ komplizierte Befestigung der Batterie am Socken auf. Außerdem ist zur Anordnung des Heizelements eine Tasche innerhalb des Sockens notwendig, die zum einen aufwendig herzustellen ist und die zum anderen zu einer deutlichen bereichsweisen Erhöhung der Materialstärke der Socke führt, die von einem Nutzer als einem Tragekomfort abträglich wahrgenommen werden könnte.

US 2009/0056107 beschreibt einen mehrschichtigen elektrischen beheizbaren Artikel, wie einen Handschuh und eine Socke, der als eine Schicht eine silberbeschichtete flexible Heizlage aufweist, die mittels einer Batterie über jeweilige elektrische Kontakte mit Energie versorgt wird.

Aufgabe der Erfindung ist es daher, eine elektrisch beheizbare Socke und eine Sockenheizanordnung zu schaffen, die einfach herzustellen und bequem zu tragen sind und eine zuverlässige Befestigung der für eine Heizung notwendigen Bestandteile gewährleisten.

Diese Aufgabe wird erfindungsgemäß durch eine elektrisch beheizbare Socke mit den Merkmalen des Anspruchs 1, eine Sockenheizanordnung mit den Merkmalen des Anspruchs 11 und ein Verfahren mit den Merkmalen des Anspruchs 14 gelöst. Vorteilhafte Weiterbildungen und Ausgestaltungen ergeben sich mit den Merkmalen der Unteransprüche.

Durch die Ausbildung mit umschlagbarer Stulpe und die entsprechende Anordnung und Ausbildung der Kontakte kann durch das Umschlagen die Batterie sicher befestigt und in eine für den Nutzer nicht störende und vorzugsweise von außen nicht sichtbare Position gebracht werden. Dadurch, dass die Anschlüsse selbst zum positionsgerechten Fixieren und zumindest teilweisen Halten der Batterie ausgebildet sind, ist nur ein flacher Aufbau gegeben und der Nutzer wird beim Tragen der Socke nicht gestört, wobei außerdem eine zuverlässige Befestigung der notwendigen Bestandteile gewährleistet ist.

Je nach Ausführungsform kann die Socke aus einer oder mehreren Materiallagen gebildet sein.

Bei zwei- oder mehrlagigen Socken weisen Fuß- und Beinteil zusätzlich zur Stulpe einen Innensocken und einen Außensocken auf, die sich in Richtung zum Fuß hin an die Stulpe anschließen. Hierdurch ist ein geschütztes Anordnen des Heizelements bei geringem Herstellungsaufwand der Socke möglich. Innen- und Außensocke gehen beide im oberen Bereich des Beinteils in die Stulpe über.

Es sind jedoch auch einlagige Socken möglich. Bei diesen ist das Heizelement zumeist auf der Innenseite der Socke aufgebracht. Nähte, die zur Befestigung des Heizelements dienen, können von außen sichtbar sein und als Designelemente dienen. Alternativ zu einer Befestigung auf der Innenseite ist jedoch auch eine Befestigung des Heizelements auf der Außenseite der Socke möglich Einlagige Socken weisen insbesondere Vorteile hinsichtlich des Fertigungsaufwands auf.

Besonders bevorzugt sind die Anschlüsse und die Stulpe so ausgebildet, dass ein Halten der Batterie ausschließlich durch die Anschlüsse sowie durch ein Andrücken über die umgeschlagene Stulpe ermöglicht wird. Somit ist eine einfache und trageleichte Anordnung der Batterie gegeben.

Vorzugsweise sind die Anschlüsse als Form- und/oder Kraftschluss bildende Kontakte ausgebildet. Eine besonders zuverlässige mechanische Anbindung der Batterie ist bei Ausbildung der Anschlüsse als Druckknopfkontakte möglich. Durch die Ausbildung als Form- und/oder Kraftschluss bildende Kontakte ist ein einfaches Abtrennen einer am Socken befestigten Batterie bzw. eines Batteriepacks möglich. Hierdurch ist ein Aufwand zum Batteriewechsel bzw. zum Abklemmen der Batterie zweck Nachladen besonders gering. Auch ein Abklemmen der Batterie zum Waschen des Sockens ist so vereinfacht.

In einer weiteren vorteilhaften Ausführungsform sind die Zuleitungen auf einem zumindest in einer, vorzugsweise in allen, Richtungen flexiblen Trägerelement angeordnet und vorzugsweise mäander-, zickzack und/oder wellenförmig aus einer Längsrichtung der Socke heraus verlaufend ausgebildet. Das Trägerelement erstreckt sich vorzugsweise nur über einen Teilbereich der Socke und ist zusätzlich zu der bzw. den Materiallagen der Socke vorgesehen. Durch das flexible, vorzugsweise dehnbare und besonders vorzugsweise textile Trägerelement wird ein besonders hoher Tragekomfort der elektrisch beheizbaren Socke erreicht. Die mäander-, zickzack oder wellenförmige Ausbildung der Zuleitungen vermeidet eine Einschränkung der Flexibilität und/oder Dehnbarkeit des Trägerelements durch die Zuleitungen, da eine Dehnung der Zuleitungen in Längsrichtung der Socke zu nur einer geringfügigen Vergrößerung der Leiterbahnlänge der Zuleitungen führt. Die Zuleitungen sind also nach dem Prinzip einer Feder dehnbar. Bevorzugt kann das Heizelement selbst mit auf dem flexiblen Trägerelement aufgebracht sein. Das Heizelement und die Zuleitungen sind dann im Bereich des flexiblen Trägerelements miteinander verbunden.

Besonders bevorzugt kann auf dem flexiblen Trägerelement im Bereich der Verbindung zwischen Heizelement und Zuleitungen ein Verstärkungselement aufgebracht sein, auf dem die Verbindung angeordnet ist. Hierdurch wird die für mechanische Beschädigungen an der anfälligsten Stelle der Leitungen, nämlich die Verbindung, zusätzlich geschützt. Das Verstärkungselement ist vorzugsweise ebenfalls flexibel, wobei besonders vorzugsweise eine Flexibilität des Verstärkungselements kleiner ist als eine Flexibilität des flexiblen Trägerelements.

Des Weiteren kann auf dem Verstärkungselement ein Einbettungsmaterial zum Umhüllen der Verbindung sowie ein die Verbindung überdeckendes Textil angeordnet sein. Das überdeckende Textil und/oder das Verstärkungselement können mit dem Einbettungsmaterial zumindest teilweise getränkt sein. Insgesamt wird so ein Laminat gebildet, durch dass ein optimaler Schutz der Verbindung erreicht wird.

Die Anschlüsse können auf einem vorzugsweise starren und/oder textilen Träger angeordnet sein, der an der Stulpe befestigt ist. Durch einen derartigen Träger weist der beheizbare Socken im Bereich der Anschlüsse eine hinreichende Festigkeit auf, um ein Wegrutschen der Anschlüsse beim Verbinden mit einer Energiequelle bzw. einer Batterie zu vermeiden. Bei Ausbildung als textiler Träger ist ein besonders einfaches Aufbringen, beispielsweise durch Aufnähen, auf dem ebenfalls textilen Material der Stulpe bei der Herstellung der Socke möglich.

In einer weiteren bevorzugten Ausführungsform ist an der Stulpe eine Lasche angebracht, die über ein Ende der Stulpe hinaussteht. Im umgeschlagenen Zustand ragt die Lasche somit in Richtung zum Fuß hin über das Ende der Stulpe hinaus. Durch die Lasche kann die Stulpe auf einfache Weise von einem umgeschlagenen Zustand zurück in einem ausgestreckten Zustand geklappt werden, so dass eine mit der Socke an den Anschlüssen verbundene Energiequelle auf einfache Art zugänglich gemacht werden kann.

In einer besonders bevorzugten Ausführungsform ist die Lasche einteilig mit dem Träger für die Anschlüsse ausgebildet. Hierdurch kann eine solche Lasche ohne jeglichen Zusatzaufwand beim Ausbilden des Trägers für die Anschlüsse mit hergestellt werden.

Eine besonders einfache Bedienbarkeit wird erreicht, wenn auf einer Innenseite der Stulpe mindestens eine Markierung für ein Steuerelement zur Steuerung der Heizleistung angeordnet ist. Nach dem Umschlagen der Stulpe liegt die Markierung somit für einen Nutzer sichtbar außen, und kann die Bedienung von an der in der Stulpe eingeschlagenen Energiequelle angebrachten Steuerelementen vereinfachen. Hierdurch können die Vorteile einer Anordnung der Energiequelle unter der umgeschlagenen Stulpe, nämlich die durch die Stulpe gegen ein Verrutschen zusätzlich abgesicherte, vor mechanischen Einflüssen geschützte und zugleich nicht sichtbare Anordnung, mit einer einfachen Bedienbarkeit kombiniert werden.

Vorzugsweise weist die Socke einen Achillessehnenschutz auf, der entweder in die Innensocke, in die Außensocke oder zwischen der Innen- und Außensocke integriert sein kann. Der Achillessehnenschutz kann beispielsweise durch eine zusätzliche Polsterung, größere Materialstärke und/oder zusätzliche Textillage gebildet werden. Durch einen solchen Achillessehnenschutz kann das Verletzungsrisiko bei Ausübung eines Wintersports, beispielsweise beim Skifahren, deutlich reduziert werden. Die Zuleitungen können bevorzugt durch diesen Achillessehnenschutz hindurch verlaufen. Hierdurch wird eine für einen Nutzer der Socke nicht wahrnehmbare Anordnung der Zuleitungen erreicht, da die Festigkeit des Sockens in diesem Bereich durch den Achillessehnenschutz ohnehin erhöht ist.

Desweiteren sind vorzugsweise Innen- und die Außensocke an mindestens zwei Stellen miteinander verbunden. Hierdurch wird ein Verdrehen der Innensocke gegenüber der Außensocke zuverlässig vermieden. Eine besonders hohe Zuverlässigkeit ergibt sich hierbei, wenn eine der Verbindungsstellen am Beinteil und eine Verbindungsstelle am Fußteil angeordnet sind. Beispielsweise können Innen- und Außensocke an bzw. direkt unter der Stulpe sowie im Zehenbereich verbunden sein.

In einer weiteren vorteilhaften Ausführungsform einer zwei- oder mehrlagigen Socke ist das mindestens eine Heizelement und/oder das Trägerelement mit Zuleitungen zwischen Innen- und Außensocke angeordnet. Hierdurch wird ein hoher mechanischer Schutz des Heizelements bzw. der Zuleitungen erreicht, wodurch eine zu erwartende Lebensdauer der Socke gesteigert werden kann.

Bei einer einlagigen Socke ist das Trägerelement mit dem darauf aufgebrachten Heizelement und den Zuleitungen bevorzugt auf der Innenseite der Socke aufgebracht. Das Heizelement und die Zuleitungen sind dabei vorzugsweise auf der zur Socke hinweisenden, d.h. vom Fuß wegweisenden Seite des Trägerelements aufgebracht. Hierdurch ist das Heizelement einerseits aufgrund seiner Anordnung auf der Innenseite der Socke mechanisch geschützt angeordnet, während andererseits aufgrund des nach Innen weisenden Trägerelements ein direkter Hautkontakt des Heizelements beim Tragen der Socke vermieden wird. Beim Tragen der Socke liegt das nach Innen weisende Trägerelement am Fuß bzw. am Bein an.

Ist das Trägerelement mit den Zuleitungen und dem Heizelement auf der Außenseite der Socke angebracht, sind die Zuleitungen und das Heizelement auf der zum Fuß weisenden Seite des Trägerelements angeordnet. Somit ist auch bei einer Anordnung des Trägerelements auf der Außenseite der Socke ein mechanischer Schutz des Heizelements gewährleistet. Das Trägerelement ist bei einer Anordnung auf der Außenseite der Socke sichtbar und kann als Designelement dienen.

Besonders einfach ist ein Aufbringen des Heizelements bzw. des Trägers mit Zuleitungen durch Nähen möglich. Um einen möglichst guten thermischen Kontakt zum Fuß hin zu erreichen ist das Heizelement bzw. der Träger mit den Zuleitungen vorzugsweise mit dem Innensocken vernäht.

Zusätzlich zu einer elektrisch beheizbaren Socke betrifft die Erfindung eine Sockenheizanordnung mit einer beheizbaren Socke, wie vorangehend beschrieben, und einem Batteriepack. Das Batteriepack weist zu den Anschlüssen der Socke korrespondierende Kontakte auf, wobei das Batteriepack über die Kontakte lösbar an der Socke befestigt ist. Durch Umschlagen der Stulpe nach dem Verbinden der Kontakte mit den Anschlüssen der Socke kann das Batteriepack unsichtbar und vor mechanischer Beanspruchung geschützt unter der Stulpe angeordnet werden.

In einer bevorzugten Ausführungsform einer Sockenheizanordnung umfasst das Batteriepack eine Steuereinheit zur Steuerung der Heizleistung, wobei die Steuereinheit einen Empfänger zum Ansteuern über eine Fernbedienung und/oder ein Eingabeelement zum Einstellen einer gewünschten Heiztemperatur aufweist. Das Eingabeelement ist bevorzugt auf derselben Seite wie die Kontakte angeordnet und vorzugsweise so positioniert, dass im umgeschlagenen Zustand der Stulpe der Bereich der Stulpe mit den Markierungen das mindestens eine Eingabeelement abdeckt.

Außerdem betrifft die Erfindung ein Verfahren zum Herstellen einer beheizbaren Socke, wobei zunächst ein Fußinnenteil, ein Beininnenteil, eine Stulpe, ein Beinaußenteil und ein Fußaußenteil in der angegebenen Reihenfolge produziert, beispielsweise gestrickt werden. Ebenso ist eine Herstellung in genau umgekehrter Reihenfolge möglich.

Insgesamt wird so ein schlauchförmiges Objekt produziert, das mindestens ein offenes Ende aufweist. Die Enden des schlauchförmigen Objekts werden hierbei durch das Fußinnenteil und das Fußaußenteil gebildet, wobei sich eine Öffnung des schlauchförmigen Objekts aus einem noch nicht fertiggestellten Zehenbereich ergibt. Mit einem "schlauchförmigen Objekt" ist hierbei nicht zwingend ein einfacher Schlauchling gemeint. Vielmehr ist eine einer Fußform nachempfundene Form des Fußinnenteils und des Fußaußenteils als besonders verteilhaft anzusehen.

Vor, während oder nach dem Fertigen des schlauchförmigen Objekts werden mindestens ein Heizelement sowie Zuleitungen auf ein flexibles Trägerelement aufgebracht. Das flexible Trägerelement mit dem Heizelement und den Zuleitungen wird nachfolgend in oder auf das schlauchförmige Objekt ein- bzw. aufgebracht. Außerdem werden Anschlüsse zum lösbaren mechanischen und elektrischen Verbinden der Socke mit einer Spannungsversorgung an der Socke angebracht.

Das schläuchförmige Objekt mit den daran angeordneten Anschlüssen und dem flexible Trägerelement mit dem Heizelement und den Zuleitungen wird anschließend derart umgestülpt, dass das Fußinnenteil im Fußaußenteil und das Beininnenteil im Beinaußenteil angeordnet werden. Hierdurch entsteht eine Socke, wobei der Zehenbereich noch offen ist. Dieser wird anschließend, beispielsweise durch Ketteln, verschlossen.

Beim Verschließen des Fußbereichs werden der Fußinnenteil und der Fußaußenteil vorzugsweise miteinander verbunden, wobei durch diese Verbindungen zusammen mit der Verbindung über die Stulpe ein Verdrehen des Innensockens gegenüber dem Außensocken verhindert wird.

Ausführungsbeispiele der Erfindung werden nachfolgend anhand der Figuren näher erläutert. Es zeigen:
- Fig. 1.: eine schematische Darstellung einer vorteilhaften Ausführungsform einer elektrisch beheizbaren Socke,
- Fig. 2.: eine Darstellung der Kontaktierung und Befestigung des Batteriepacks,
- Fig. 3.: eine perspektivische Ansicht des Batteriepacks von schräg vorne,
- Fig. 4.: eine perspektivische Ansicht des Batteriepacks von schräg hinten,
- Fig. 5.: eine Darstellung eines Sockenrohlings nach einem ersten Verfahrensschritt einer vorteilhaften Ausführungsform des erfindungsgemäßen Verfahrens und
- Fig. 6.: eine Aufsicht auf ein Untermaterial mit darauf aufgebrachtem Heizelement und Zuleitungen.

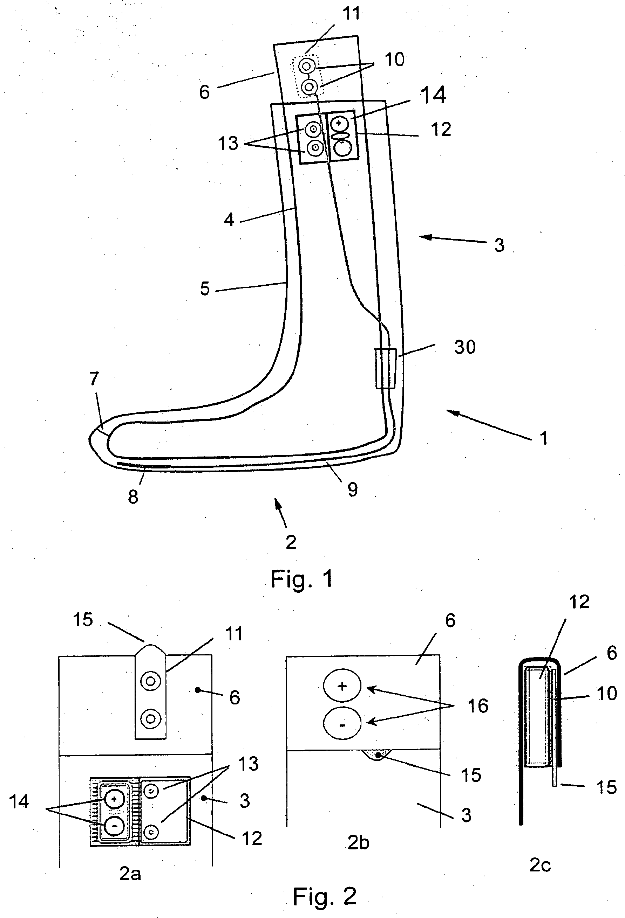

In Fig. 1 ist eine elektrisch beheizbare Socke 1 dargestellt, die einen Fußbereich 2 und einen Beinbereich 3 aufweist. Die Socke 1 umfasst eine Innensocke 4 und eine Außensocke 5, wobei Innen- und Außensocke 4, 5 an ihrer Oberkante in eine Stulpe 6 übergehen und durch diese miteinander verbunden sind. Im Bereich der Zehen ist eine weitere Verbindung 7 zwischen Innensocke 4 und Außensocke 5 vorgesehen, durch die ein Verdrehen der Innensocke 4 relativ zur Außensocke 5 zuverlässig verhindert wird. Ebenfalls im Bereich der Zehen angeordnet ist ein Heizelement 8, das über Zuleitungen 9 mit Druckknopfkontakten 10 verbunden ist. Die Zuleitungen verlaufen ausgehend vom Heizelement 8 zunächst unterhalb des Fußes gerade bis zur Ferse und sind dort fixiert. Ausgehend von der Ferse durchlaufen die Zuleitungen einen Achillessehnenschutz 30 und werden oberhalb von diesem seitlich vom Unterschenkel zu den als Druckknopfkontakten 10 ausgebildeten Anschlüssen geführt.

Die Druckknopfkontakte 10 sind im Bereich der Stulpe 6 angeordnet und an dieser mittels eines aufgenähten textilen Trägers 11 befestigt. Unterhalb der Stulpe ist ein Batteriepack 12 dargestellt. Das Batteriepack ist hierbei an der Position gezeigt, an der es bei umgeschlagener Stulpe 6 fixiert wird. Das Batteriepack 12 weist Druckknopfkontakte 13 auf, die zu denDruckknopfkontakten 10 an der Stulpe 6 korrespondieren und dazu ausgebildet sind, eine formschlüssige Verbindung mit diesen einzugehen. Außerdem umfasst das Batteriepack 12 Steuerelemente 14, die mit einem im Batteriepack 12 integrierten Steuermodul zur Regulierung der Heizleistung verbunden sind.

Das Prinzip der Befestigung des Batteriepacks ist in Fig. 2 im Detail dargestellt. Die Fig. 2a stellt das obere Ende des elektrisch beheizbaren Sockens 1 bei aufgeklapptem Bündchen bzw. Stulpe dar. Das Batteriepack ist knapp unterhalb der Stulpe 6 eingezeichnet, wobei es üblicherweise bei aufgeklappter Stulpe 6 nicht fixiert ist und somit zur Erläuterung eingezeichnet ist. Falls gewünscht kann jedoch die Unterseite des Batteriepacks mit einem Klettverschluss versehen werden, der an der Socke anhaftet.

In Fig. 2b ist das obere Ende des Sockens 1 bei umgeschlagener Stulpe dargestellt. Das nach unten zeigende Ende der Stulpe 6 wird von einer Lasche 15 überragt, die, wie in Fig. 2a zu erkennen, einteilig mit dem Träger 11 ausgebildet ist. In umgeschlagenem Zustand der Stulpe 6 sind die Druckknopfkontakte 10 auf dem Träger 11 mit den Druckknopfkontakten 13 des Batteriepacks 12 verbunden. Hierdurch wird eine Fixierung des Batteriepacks 12 erreicht. Eine weitere Stabilisierung der Lage des Batteriepacks 12 ergibt sich dadurch, dass ein Umfang der Stulpe 6 im umgeklappten Zustand aufgrund des darunter angeordneten Batteriepacks 12 gegenüber einem Umfang in nicht umgeschlagenen Zustand vergrößert ist, wobei die Stulpe einer hierdurch gegebenen Dehnung mit einer Kraft entgegenwirkt, wodurch das Batteriepack 12 in Richtung Bein angedrückt wird.

Im umgeschlagenen Zustand der Stulpe 6 zeigen Markierungen 16, die auf einer Innenseite der Stulpe 6 aufgebracht sind, nach außen und sind somit für einen Nutzer sichtbar. Diese Markierungen liegen auf den Eingabeelementen 14, die beispielsweise als Druckkontakte oder Taster ausgebildet sein können. Durch die Markierungen 16 sind die Positionen der Eingabeelemente 14 für einen Nutzer sichtbar gekennzeichnet, wodurch ein zuverlässiges Betätigen der Eingabeelemente 14 des an sich verdeckten Batteriepacks 12 ohne vorheriges Aufklappen der Stulpe 6 (diese wird oft auch als Bündchen bezeichnet) möglich wird.

In Fig. 2c ist das obere Ende des elektrisch beheizbaren Sockens 1 im Schnitt dargestellt, wobei in dieser Ansicht die Funktionsweise der Lasche 15 besonders gut zu erkennen ist. Diese steht aufgrund der sich durch das Batteriepack 12 ergebenden, nach vorne versetzten Anordnung frei und kann leicht von einem Nutzer gegriffen werden. Hierdurch wird ein Aufklappen des Bündchens 6, beispielsweise zum Wechsel oder zum Nachladen des Batteriepacks 12 erheblich vereinfacht.

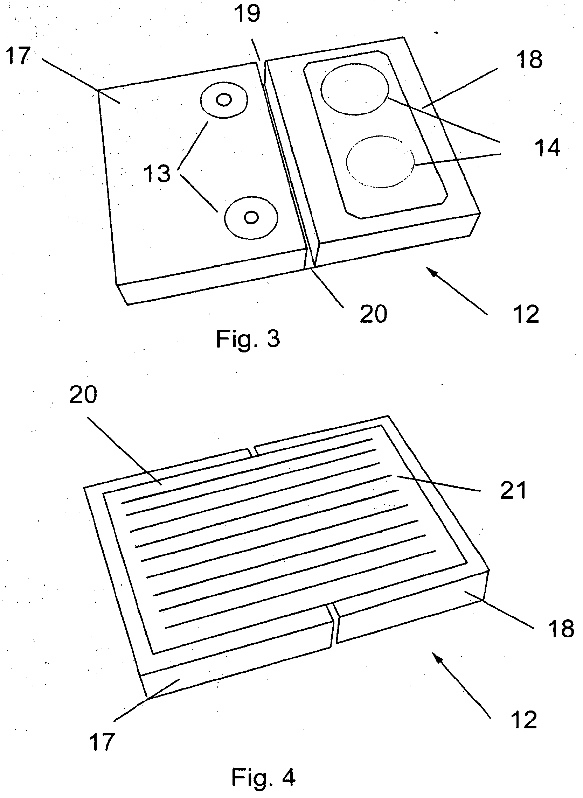

Das Batteriepack 12 ist in einer perspektivischen Ansicht von schräg oben in Fig. 3 genauer dargestellt. Das Batteriepack 12 ist zweiteilig mit einem ersten Teil 17 und einem zweiten Teil 18 ausgebildet. An dem ersten Teil 17 sind die Druckknopfkontakte 13 angeordnet, während der zweite Teil 18 die Eingabeelemente 14 aufweist. Zwischen beiden Teilen 17, 18 ist eine Lücke 19, wobei eine Verbindung beider Teile über eine flexible Schicht 20 gewährleistet ist. Sowohl die äußere Umhüllung der beiden Teile 17, 18 als auch die Schicht 20 können aus Silikon oder einem anderen ähnlich flexiblen Kunststoff bestehen. Durch die Lücke 19 zwischen beiden Teilen 17, 18 kann die Schicht 20 als ein Filmscharnier dienen, wodurch ein Verkippen beider Teile 17, 18 relativ zueinander ermöglicht wird. Dieses Verkippen ermöglicht eine verbesserte Anpassung an eine Form des Beins eines Nutzers der elektrisch beheizbaren Socke 1 und führt somit zu einer deutlichen Erhöhung des Tragekomforts.

In Fig. 4 ist das Batteriepack 12 in einer perspektivischen Ansicht auf die Rückseite dargestellt, wobei in dieser Ansicht zu erkennen ist, dass die Rückseite der Schicht 20 Riffel 21 aufweist. Diese dienen einer Erhöhung der Haftreibung zwischen dem Batteriepack 21 und dem Beinbereich 3 der Socke 1 und reduzieren somit weiter das Risiko eines Verrutschens des Batteriepacks 12.

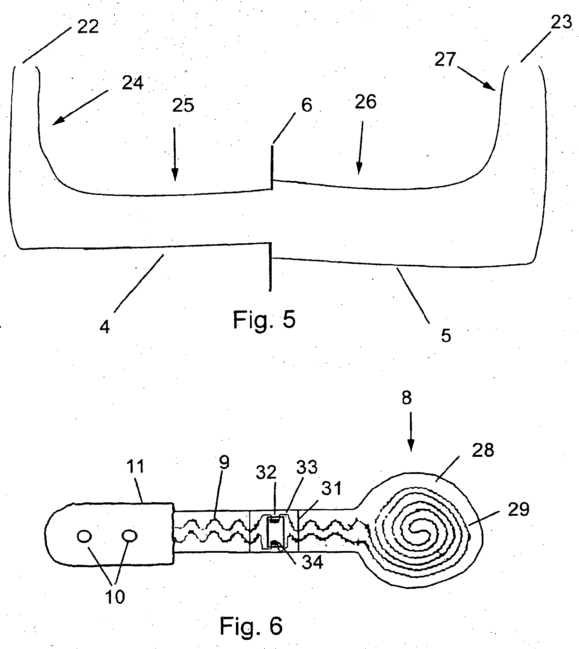

In Fig. 5 ist ein Rohling einer elektrisch beheizbaren Socke nach einem ersten Verfahrensschritte eines Verfahrens zur Herstellung einer elektrisch beheizbaren Socke dargestellt. Der Rohling ist im Wesentlichen schlauchförmig ausgebildet, und weist einen Innenfußbereich 24, einen Innenbeinbereich 25, eine Stulpe 6, einen Außenbeinbereich 26 und einen Außenfußbereich 27 auf. Die verschiedenen Bereiche werden in der genannten Reihenfolge oder genau umgekehrt hergestellt. Es wird als an einem Ende des schlauchförmig ausgebildeten Rohlings mit der Herstellung begonnen und dieser wird dann durchgehend in einer Richtung gefertigt. Die aus Innenfuß- und Innenbeinbereich 24, 25 bestehende Innensocke 4 sowie die aus Außenfuß- 27 und Außenbeinbereich 26 bestehende Außensocke 5 sind jeweils einer Fußform entsprechend ausgebildet. Sowohl Innensocke 4 als auch Außensocke 5 weisen zu diesem Zeitpunkt des Verfahrens eine Öffnung 22, 23 im Bereich der Zehen auf.

In Fig. 6 ist ein auf einem Untermaterial 28 aufgebrachtes Heizelement 8 dargestellt. Das Heizelement 8 weist einen Heizdraht 29 oder eine Heizlitze auf, der bzw. die spiralförmig verläuft. Eine Heizlitze ist hierbei aufgrund ihrer Flexibilität bevorzugt. Der Heizdraht 29 bzw. die Heizlitze ist mit einem flexiblen Kunststoff, beispielsweise PVC, ummantelt und somit isoliert. In dem dargestellten Beispiel ist der Heizdraht 29 als eine Doppelspirale ausgebildet, d.h. der Heizdraht verläuft, ausgehend von einem Ende, als Spirale nach innen und verläuft dann weiter spiralförmig zurück nach außen zum anderen Ende. Der Heizdraht 29 könnte jedoch auch als eine einfache Spirale, die dann zweiadrig ausgebildet ist, verlaufen. Es können also eine hin- und eine zurücklaufende Ader eines Heizdrahtes in einem gemeinsamen Mantel angeordnet sein. Selbstverständlich können auch andere Komponenten für das Heizelement, wie Leiterbahnen oder andere Widerstandsflächen, verwendet werden.

Die Enden des Heizdrahts 29 sind mit Zuleitungen 9 verbunden, die wellenförmig ausgebildet sind. Die Verbindung ist auf einem Verstärkungselement 32 angeordnet. Zum Verbinden wurden Enden 33 des Heizdrahts 29 bzw. der Zuleitungen 9 auf Kontaktbereiche 34 des Verstärkungselements 32 aufgelötet. Die Enden 33 werden hierbei so zum Kontaktbereich 34 geführt, dass sie seitlich zu einer Längsrichtung des Sockens, d.h. in Richtung einer sich durch eine Abrollbewegung beim Gehen ergebende Biegeachse, verlaufen. Hierdurch werden die Enden bei einer beim Gehen entstehenden Abrollbewegung um ihre Achse verdreht statt geknickt. Dies verringert signifikant ein Ausfallrisiko durch ein Ablösen der Verbindung.

Das Verstärkungselement 32 wird zur weiteren Stabilisierung mit einem Textil 31 überdeckt. Vor dem Aufbringen des Textils 31 wurde ein nicht dargestelltes Einbettungsmaterial, beispielsweise ein Silikon, auf das Verstärkungselement aufgebracht. Das Einbettungsmaterial umhüllt die Verbindung und stabilisiert diese hierdurch zusätzlich. Zusätzlich saugt sich das Verstärkungselement und das Textil mit dem Einbettungsmaterial voll, wodurch sich eine besonders gute Anpassung der Materialeigenschaften an das Einbettungsmaterial ergibt.

Insgesamt erhält man so ein flexibles Laminat, dass die Verbindung entlastet und Zug sowie Biegekräfte von ihr ableitet.

Die wellenförmige Ausbildung der Zuleitungen 9 (in gleicher Weise ist eine mäanderförmige oder zickzackförmige Ausbildung der Zuleitungen möglich) führt dazu, dass eine Dehnung der Zuleitungen 9 in ihrer Längsrichtung mit geringer Kraft und ohne Beschädigung der Zuleitungen 9 möglich ist. An das Untermaterial 28 mit dem Heizelement 8 und den Zuleitungen 30 schließt sich der Träger 11 mit den Druckknopfkontakten 10 an. Das dargestellte Untermaterial mit dem Heizelement 8 und den Zuleitungen 9 wird gefertigt, indem der Heizdraht 29 und die Zuleitungen 9 auf das Untermaterial 28 aufgenäht werden. Anschließend wird das Untermaterial 28 mit den Leitungen durch die Öffnung 22 in den Innensocken 4 eingeführt und mit diesem vernäht. Nachfolgend wird der Innensocken 4 umgestülpt, so dass er im Außensocken 5 angeordnet wird, wobei das auf dem Innensocken 4 aufgenähte Untermaterial 28 zwischen Innensocken 4 und Außensocken 5 positioniert ist. Anschließend werden die Öffnungen 22, 23 verschlossen. Dies ist beispielsweise durch Kettell möglich, wobei zugleich der Innensocken 4 mit dem Außensocken 5 im Bereich der Zehen verbunden wird.

## Patentansprüche

1. Elektrisch beheizbare Socke mit einem Fußteil (2) und einem daran anschließenden Beinteil (3), wobei mindestens ein Heizelement (8) am Fußteil (2) angeordnet ist, das über Zuleitungen (9) mit Anschlüssen (10) für eine eine Batterie aufweisende Spannungsversorgung verbunden ist, wobei die Anschlüsse (10) im oberen Bereich des Beinteils angeordnet sind, **dadurch gekennzeichnet, dass** am oberen Ende des Beinteils (3) eine umschlagbare Stulpe (6) angeordnet ist, wobei die Anschlüsse (10) an der im umgeschlagenen Zustand innenliegenden Seite der Stulpe (6) befestigt sind und ausgebildet sind, die Batterie im umgeschlagenen Zustand der Stulpe (6) positionsgerecht zu fixieren und zumindest teilweise zu halten.

2. Socke nach Anspruch 1, **dadurch gekennzeichnet, dass** Fuß- und Beinteil (2, 3) unterhalb der Stulpe (6) eine Innensocke (4) und eine Außensocke (5), die miteinander verbunden sind, aufweisen.

3. Socke nach einem der vorangehenden Ansprüche, **dadurch gekennzeichnet, dass** die Anschlüsse (10) als Form- und/oder Kraftschluss bildende Kontakte, insbesondere als Druckknopfkontakte ausgebildet sind.

4. Socke nach einem der vorangehenden Ansprüche, **dadurch gekennzeichnet, dass** die Zuleitungen (9) auf einem flexiblen Trägerelement (28) angeordnet und vorzugsweise mäander-, zickzack und/oder wellenförmig aus einer Längsrichtung der Socke heraus verlaufend ausgebildet sind.

5. Socke nach einem der vorangehenden Ansprüche, **dadurch gekennzeichnet, dass** die Anschlüsse (10) auf einem, vorzugsweise flexiblen und/oder textilen Träger (11) angeordnet sind, der an der Stulpe (6) befestigt ist.

6. Socke nach einem der vorangehenden Ansprüche, **dadurch gekennzeichnet, dass** an der Stulpe (6) eine Lasche (15) angebracht ist, welche im umgeschlagenen Zustand über das Ende der Stulpe (6) hinaussteht.

7. Socke nach Anspruch 6, **dadurch gekennzeichnet, dass** die Lasche (15) einteilig mit dem Träger (11) für die Anschlüsse (10) ausgebildet ist.

8. Socke nach einem der vorangehenden Ansprüche, **dadurch gekennzeichnet, dass** auf einer Innenseite der Stulpe (6) mindestens eine Markierung (16) für ein Steuerelement (14) zur Steuerung der Heizleistung angeordnet ist.

9. Socke nach einem der Ansprüche 2 bis 8, **dadurch gekennzeichnet, dass** die Innen- und die Außensocke (4, 5) an mindestens zwei Stellen, wovon vorzugsweise mindestens eine Stelle im Beinteil (3) und eine im Fußteil (2) liegt, miteinander verbunden sind.

10. Socke nach einem der Ansprüche 2, bis 9, **dadurch gekennzeichnet, dass** das mindestens eine Heizelement (8) und/oder das Trägerelement (28) mit Zuleitungen zwischen Innen- und Außensocke (4, 5) angeordnet und zumindest mit dem Innensocken (4) vorzugsweise durch Nähen verbunden ist.

11. Sockenheizanordnung mit einer beheizbaren Socke nach einem der vorangehenden Ansprüche sowie mindestens einem Batteriepack (12, wobei das Batteriepack (12) zu den Anschlüssen (10) der Socke korrespondierende Kontakte (13) aufweist und wobei das Batteriepack (12) über die Kontakte (13) lösbar an der Socke befestigt ist.

12. Sockenheizanordnung nach Anspruch 11, **dadurch gekennzeichnet, dass** das Batteriepack (12) eine Steuereinheit (14, 18) zur Steuerung der Heizleistung umfasst, wobei die Steuereinheit (14, 18) einen Empfänger zum Ansteuern über eine Fernbedienung oder ein Eingabeelement (14) zum Einstellen einer gewünschten Heizleistung aufweist.

13. Sockenheizanordnung nach Anspruch 12, **dadurch gekennzeichnet, dass** das Eingabeelement (14) auf derselben Seite des Batteriepacks (12) wie die Kontakte (13) angeordnet ist.

14. Verfahren zum Herstellen eines beheizbaren Sockens, mit den Schritten:
- Herstellen eines Fußinnenteils (24), eines Beininnenteils (25), einer Stulpe (6), eines Beinaußenteils (26) und eines Fußaußenteils (27) in der angegebenen oder umgekehrten Reihenfolge als ein einteiliges schlauchförmiges Objekt mit mindestens einem offenen Ende (22, 23),
- Aufbringen eines Heizelements (8) sowie von Zuleitungen (9) auf ein flexibles Trägerelement
- Aufbringen des flexiblen Trägerelements (28) mit dem Heizelement (8) und den Zuleitungen (9) in oder auf das schlauchförmig Objekt, **gekennzeichnet durch**
- Anbringen von Anschlüssen (10) an der Stulpe (6) zum lösbaren mechanischen und elektrischen Verbinden der Socke mit einer Spannungsversorgung,
- teilweises Umstülpen des schlauchförmigen Objekts, sodass ein Socken gebildet wird, wobei der Fußinnenteil (24) mit dem Fußaußenteil (27) einen Fußteil (2) des Sockens und der Beininnenteil (25) mit dem Beinaußenteil (26) und der Stulpe (6) einen Beinteil (3) des Sockens bildet,
- Verschließen des mindestens einen offenen Endes.

15. Verfahren nach Anspruch 14, **dadurch gekennzeichnet, dass** der Fußinnenteil (24) und der Fußaußenteil (27) beim Verschließen des mindestens einen offenen Endes (22, 23) miteinander verbunden, beispielsweise verkettelt werden.

## Claims

1. Electrically heatable sock having a foot part (2) and a leg part (3) abutting thereon, at least one heating element (8) being disposed on the foot part (2) and being connected via supply lines (9) to terminals (10) for a voltage supply which has a battery, the terminals (10) being disposed in the upper region of the leg part (3), **characterised in that** a cuff (6) which can be folded over is disposed at the upper end of the leg part (3), the terminals (10) being attached and formed on the side of the cuff (6) situated inside in the folded-over state in order to fix the battery in the correct position in the folded-over state of the cuff (6) and to retain it at least partially.

2. Sock according to claim 1, **characterised in that** foot- and leg part (2, 3) underneath the cuff (6) have an inner sock (4) and an outer sock (5) which are connected to each other.

3. Sock according to one of the preceding claims, **characterised in that** the terminals (10) are configured as contacts which constitute a form-fit and/or frictional connection, in particular as push-button contacts.

4. Sock according to one of the preceding claims, **characterised in that** the supply lines (9) are disposed on a flexible carrier element (28) and are configured to extend preferably in a meandering, zigzag and/or undulating shape from a longitudinal direction of the sock.

5. Sock according to one of the preceding claims, **characterised in that** the terminals (10) are disposed on a preferably flexible and/or textile carrier (11) which is attached to the cuff (6).

6. Sock according to one of the preceding claims, **characterised in that** a tab (15) which protrudes beyond the end of the cuff (6) in the folded-over state is fitted on the cuff (6).

7. Sock according to claim 6, **characterised in that** the tab (15) is configured in one piece with the carrier (11) for the terminals (10).

8. Sock according to one of the preceding claims, **characterised in that** at least one marking (16) for a control element (14) for controlling the heating power is disposed on an inner side of the cuff (6).

9. Sock according to one of claims 2 to 8, **characterised in that** the inner-and the outer sock (4, 5) are connected to each other at at least two places, preferably at least one place of which is situated in the leg part (3) and one in the foot part (2).

10. Sock according to one of claims 2 to 9, **characterised in that** the at least one heating element (8) and/or the carrier element (28) is disposed with supply lines between inner and outer sock (4, 5) and is connected at least to the inner sock (4) preferably by sewing.

11. Sock heating arrangement having a heatable sock according to one of the preceding claims and also at least one battery pack, the battery pack (12) having contacts (13) which correspond to the terminals (10) of the sock and the battery pack (12) being attached detachably to the sock via the contacts (13).

12. Sock heating arrangement according to claim 11, **characterised in that** the battery pack (12) includes a control unit (14, 18) for controlling the heating power, the control unit (14, 18) having a receiver for actuation via a remote control or an input element (14) for setting a desired heating power.

13. Sock heating arrangement according to claim 12, **characterised in that** the input element (14) is disposed on the same side of the battery pack (12) as the contacts (13).

14. Method for producing a heatable sock, with the steps:
- production of a foot inner part (24), a leg inner part (25), a cuff (6), a leg outer part (26) and a foot outer part (27) in the indicated or reverse sequence as a one-piece tubular object with at least one open end (22, 23),
- application of a heating element (8) and also supply lines (9) on a flexible carrier element,
- application of the flexible carrier element (28) with the heating element (8) and the supply lines (9) in or on the tubular object, **characterised by**
- fitting of terminals (10) on the cuff (6) for detachable mechanical and electrical connection of the sock to a voltage supply,
- partial turning over of the tubular object so that a sock is formed, the foot inner part (24) with the foot outer part (27) forming a foot part (2) of the sock and the leg inner part (25) with the leg outer part (26) and the cuff (6) forming a leg part (3) of the sock,
- closing of the at least one open end.

15. Method according to claim 14, **characterised in that** the foot inner part (24) and the foot outer part (27) are connected to each other by closing the at least one open end (22, 23), for example linking.

## Revendications

1. Chaussette chauffante électrique avec une partie de pied (2) et une partie de jambe (3) qui y est raccordée, au moins un élément chauffant (8) étant disposé sur la partie de pied (2), qui est relié, par l'intermédiaire de câbles d'alimentation (9) à des raccordements (10) pour une alimentation en tension comprenant une batterie, les raccordements (10) étant disposés dans la partie supérieure de la partie de jambe, **caractérisée en ce que**, au niveau de l'extrémité supérieure de la partie de jambe (3), se trouve une manchette rabattable (6), les raccordements (10) étant fixés sur le côté intérieur, dans l'état rabattu, de la manchette (6) et conçus pour fixer et maintenir au moins partiellement la batterie en position dans l'état rabattu de la manchette (6).

2. Chaussette selon la revendication 1, **caractérisée en ce que** la partie de pied et la partie de jambe (2, 3) comprennent, en dessous de la manchette (6), une chaussette interne (4) et une chaussette externe (5) qui sont reliées entre elles.

3. Chaussette selon l'une des revendications précédentes, **caractérisée en ce que** les raccordements (10) sont conçus comme des contacts par complémentarité de forme ou par friction, plus particulièrement comme des contacts à boutons poussoir.

4. Chaussette selon l'une des revendications précédentes, **caractérisée en ce que** les câbles d'alimentation (9) sont disposés sur un élément de support flexible (28) et sont conçus de préférence avec une forme de méandres, de zigzag et/ou avec des ondulations et de façon à s'étendre à partir d'une direction longitudinale de la chaussette.

5. Chaussette selon l'une des revendications précédentes, **caractérisée en ce que** les raccordements (10) sont disposés sur un support (11) de préférence flexible et/ou textile qui est fixé à la manchette (6).

6. Chaussette selon l'une des revendications précédentes, **caractérisée en ce que**, sur la manchette (6) est prévue une patte (15) qui dépasse de l'extrémité de la manchette (6) dans l'état rabattu.

7. Chaussette selon la revendication 6, **caractérisée en ce que** la patte (15) est conçu d'une seule pièce avec le support (11) pour les raccordements (10).

8. Chaussette selon l'une des revendications précédentes, **caractérisée en ce que**, à l'intérieur de la manchette (6), se trouve au moins un marquage (16) pour un élément de commande (14) pour la commande de la puissance électrique.

9. Chaussette selon l'une des revendications 2 à 8, **caractérisée en ce que** la chaussette interne et la chaussette externe (4, 5) sont reliées entre elles au niveau d'au moins deux endroits, dont de préférence au moins un endroit dans la partie de jambe (3) et un dans la partie de pied (2).

10. Chaussette selon l'une des revendications 2 à 9, **caractérisée en ce que** l'au moins un élément chauffant (8) et/ou l'élément de support (28) avec des câbles d'alimentation est disposé entre la chaussette interne et la chaussette externe (4, 5) et est relié au moins avec la chaussette interne (4), de préférence par des coutures.

11. Dispositif de chauffage de chaussette sur une chaussette chauffante selon l'une des revendications précédentes ainsi qu'au moins un bloc de batterie (12), le bloc de batterie (12) comprenant, au niveau des raccordements (10) de la chaussette, des contacts (13) correspondants et le bloc de batterie (12) étant fixé de manière amovible à la chaussette par l'intermédiaire des contacts (13).

12. Dispositif de chauffage de chaussette selon la revendication 11, **caractérisé en ce que** le bloc de batterie (12) comprend une unité de commande (14, 18) pour la commande de la puissance de chauffage, l'unité de commande (14, 18) comprenant un récepteur pour la commande par l'intermédiaire d'une télécommande ou un élément d'entrée (14) pour le réglage d'une puissance de chauffage souhaitée.

13. Dispositif de chauffage de chaussette selon la revendication 12, **caractérisé en ce que** l'élément d'entrée (14) est disposé sur le même côté du bloc de batterie (12) que les contacts (13).

14. Procédé de fabrication d'une chaussette chauffante, comprenant les étapes suivantes :
- fabrication d'une partie interne de pied (24), d'une partie interne de jambe (25), d'une manchette (6), d'une partie externe de jambe (26) et d'une partie externe de pied (27) dans l'ordre indiqué ou inversement sous la forme d'un objet tubulaire d'une seule pièce avec au moins une extrémité ouverte (22, 23),
- montage d'un élément chauffant (8) ainsi que de câbles d'alimentation (9) sur un élément de support flexible,
- montage de l'élément de support flexible (28) avec l'élément chauffant (8) et les câbles d'alimentation (9) ou sur l'objet tubulaire, **caractérisé par**
- le montage de raccordements (10) sur la manchette (6) pour une liaison mécanique et électrique amovible de la chaussette avec une alimentation en tension,
- le retournement partiel de l'objet tubulaire, de façon à former une chaussette, la partie interne de pied (24) formant avec la partie externe de pied (27) une partie de pied (2) de la chaussette et la partie interne de jambe (25) formant avec la partie externe de pied (26) et la manchette (6) une partie de jambe (3) de la chaussette,
- la fermeture de l'au moins une extrémité ouverte.

15. Procédé selon la revendication 14, **caractérisé en ce que** la partie interne de pied (24) et la partie externe de pied (27) sont reliées entre elles, par exemple maillées, lors de la fermeture de l'au moins une extrémité ouverte (22, 23).
